# EUROPEAN PATENT APPLICATION

(11) **EP 4 327 770 A1**
(43) Date of publication of application: **28.02.2024**
(21) Application number: 22791876.0
(22) Date of filing: 04.03.2022
(51) Int. Cl.: A61B 90/00, A61C 5/90, A61N 7/00, A61N 1/30, A61N 1/04, A61B 18/00

(54) **DEVICE FOR ORAL INSERTION FOR CONTROLLING ENERGY TRANSFER CHARACTERISTICS**

(30) Priority: 22.04.2021 KR 20210052574; 24.05.2021 KR 20210066256; 25.10.2021 KR 20210142866
(71) Applicant: Rah, Gong-Chan, Seoul 06040 (KR)
(72) Inventor: Rah, Gong-Chan, Seoul 06040 (KR)
(74) Representative: Dehns
(86) International application number: PCT/KR2022/003114
(87) International publication number: WO 2022/225176

(57) **Abstract**

Disclosed is an oral balloon comprising: an eleventh fluid expansion part; a twelfth fluid expansion part; as a fluid bag, a fluid bag formed inside the eleventh fluid expansion part and the twelfth fluid expansion part; and a fluid injection port formed for the injection of a fluid into the fluid bag.

## Description

### TECHNICAL FIELD

The present invention relates to a device for oral insertion, that is, an energy transfer characteristic control tube that is insertable into an oral cavity. More particularly, the present invention relates to an energy transfer characteristic control tube that is insertable into an oral cavity for preventing pain from being applied to a skin or bone tissue at an unwanted position due to heat accumulation or vibration of an ultrasonic wave when performing an ultrasonic or RF treatment that applies a stimulation to an inner layer of epidermis using an ultrasonic wave or RF.

### BACKGROUND ART

Various ultrasonic treatments such as Ulthera are being performed to add elasticity to a skin or to increase the elasticity of the skin to a previous level.

This treatment that restores or increases the elasticity of the skin by using an ultrasonic wave induces regeneration of cells having new property by applying an ultrasonic stimulation to an inner layer of epidermis in an area in which the elasticity is to be restored or increased, thereby increasing the elasticity of the skin.

In general, an ultrasonic wave used in this treatment is the same as that used in SONAR. Vibration may be applied to the a treated area of a patient in a certain circumstance due to a natural frequency of the ultrasonic wave depending on each treatment and a treatment type of an operator. Here, the treatment has a problem in that the patient feels pain due to the vibration transmitted to the skin or bone tissue.

Thus, when performing the treatment (mainly, for a face) for restoring or increasing the elasticity of the skin by using the ultrasonic wave, a device that is hygienic and easily installed on a body of the patient is demanded to reduce pain and suffering of the patient during the treatment.

Also, a device for preventing the pain of the patient due to thermal energy transfer during other treatments using a RF (Radiofrequency) wave instead of the ultrasonic wave, i.e., a device that prevents thermal energy from being accumulated at a desired position, is demanded. There is another treatment called Thermage that uses the RF (Radiofrequency) wave.

The related prior art is Korea Patent Registration No. 10-1401133. According to the prior art, a microneedle is inserted into an epidermal layer of a skin to cause intentional injury in a dermis layer. While high frequency output and ultrasonic vibration are performed through the microneedle, high energy is transmitted to the dermis layer, and a skin regeneration inducing effect through partial (about 9%) necrosis is generated. Although the prior art induces the necrosis of the skin and then allows the skin to have elasticity during the regeneration process. However, the prior art does not disclose a method for improving a condition of a treatment subject (patient) that directly receives vibration or heat of the ultrasonic wave during the treatment.

### DISCLOSURE OF THE INVENTION

### TECHNICAL PROBLEM

The present invention provides a device for solving a problem such as pain and suffering of a patient during treatment when performing the above-described treatment (mainly, for a facial area) for restoring or increasing elasticity of a skin by using conventional ultrasonic wave or RF.

Specifically, by including an energy transfer characteristic control tube installed in an oral cavity of a treatment subject (patient), ultrasonic vibration transmitted to a skin of the patient is prevented from being transmitted to bone tissues such as gums or cheekbones, and thermal energy accumulation at an unwanted area due to the RF wave is prevented.

### TECHNICAL SOLUTION

Embodiments of the present invention provide an oral balloon including: a fluid bag to which fluid is injected; and a fluid injection port configured to inject fluid into the fluid bag.

In an embodiment, the oral balloon may further include: an eleventh fluid expansion part; a twelfth fluid expansion part; and a bridge part configured to connect the eleventh fluid expansion part and the twelfth fluid expansion part to each other, wherein the fluid bag is formed by the eleventh fluid expansion part and the twelfth fluid expansion part.

In an embodiment, the fluid bag may have an integrated shape formed over the eleventh fluid expansion part, the twelfth fluid expansion part, and the bridge part.

In an embodiment, the bridge part, the fluid injection port may be formed in the bridge part.

In an embodiment, the oral balloon may further include a fluid bag separation gap, and when the fluid bag is filled with fluid, the fluid bag may have a shape restricted by the fluid bag separation gap.

In an embodiment, the fluid bag separation gap may be made of a synthetic resin.

In an embodiment, the oral balloon may further include an insertion guide part connected to the fluid expansion part that forms the fluid bag, in which an implant configured to insert the oral balloon into an oral cavity may be formed in the insertion guide part.

In an embodiment, the implant may substantially maintain a shape of the oral balloon when the oral balloon is inserted into the oral cavity.

In an embodiment, the implant may include at least one of fluid, a synthetic resin, and metal.

In an embodiment, a pocket having an inlet formed toward a central portion of the oral balloon may be formed in each of the eleventh fluid expansion part and the twelfth fluid expansion part. Here, the central portion of the oral balloon may represent a center of gravity of the oral balloon.

In other embodiments of the present invention, a method for installing the oral balloon in a space between a face and an oral cavity of a patient includes: arranging a fluid bag into a mouth of the patient; and expanding the fluid bag by injecting fluid through the fluid injection port after the arranging of the fluid bag.

In other embodiments of the present invention, a method for installing the oral balloon in a space between a face and an oral cavity of a patient includes: expanding the fluid bag by injecting fluid through the fluid injection port; and arranging the fluid bag into a mouth of the patient after the expanding of the fluid bag.

In other embodiments of the present invention, an oral balloon includes: a second chamber (220) to which fluid is injected; a first chamber (210) arranged to surround a portion of an edge of the second chamber; a third chamber (230) filled with fluid; and a fluid movement passage (240) configured to receive the fluid from the third chamber and provide the fluid to the second chamber, in which the first chamber is filled with fluid, and when pressure is applied to the third chamber, the fluid contained in the third chamber is supplied to the second chamber through the fluid movement passage to expand the second chamber

In an embodiment, the first chamber and the second chamber may be separated from each other, the first chamber and the third chamber may be separated from each other, and the first chamber and the third chamber may be connected through the fluid movement passage and the second chamber.

In an embodiment, a first point (251) of a first surface of the second chamber and a second point (252) of a second surface of the second chamber may be bonded to each other, and a three-dimensional shape of the second chamber, which is formed when the second chamber is expanded, may be determined by the bonding of the first point and the second point.

In an embodiment, the fluid movement passage may have one end connected to a portion of an edge of the second chamber, which is not surrounded by the first chamber, and the other end connected to a point on an edge of the third chamber.

In other embodiments of the present invention, a method for manufacturing the oral balloon includes: overlapping a first sheet and a second sheet; performing thermal bonding to form the first chamber, the second chamber, the third chamber, and the fluid movement passage except for the fluid injection part configured to inject fluid to the first chamber and the third chamber; injecting fluid through the fluid injection part formed in each of the first chamber and the third chamber; and performing thermal bonding to block the fluid injection part of the first chamber and the fluid injection part of the third chamber.

In an embodiment, the performing of the thermal bonding may include thermally bonding a first point of the first sheet of the second chamber and a second point of the second sheet of the second chamber.

In an embodiment, the first sheet and the second sheet may be made of a synthetic resin.

In other embodiments of the present invention, an oral balloon includes a fluid bag (13) formed in each of a first fluid expansion part (11), a second fluid expansion part (12), a third fluid expansion part (21), and a fourth fluid expansion part (22) and a fluid injection port (14) for injecting fluid to the fluid bag.

In an embodiment, the oral balloon may further include a bridge portion (19) connecting the first fluid expansion part, the second fluid expansion part, the third fluid expansion part, and the fourth fluid expansion part to each other, and the fluid bag may have an integrated shape formed over the first fluid expansion part, the second fluid expansion part, the third fluid expansion part, the fourth fluid expansion part, and the bridge part.

In an embodiment, the fluid injection port may be formed in the bridge part.

In an embodiment, the first fluid expansion part may be disposed on a right upper end of a space between a face and an oral cavity of the patient, the second fluid expansion part may be disposed on a right lower end of the space between the face and the oral cavity of the patient, the third fluid expansion part may be disposed on a left upper end of the space between the face and the oral cavity of the patient, and the fourth fluid expansion part may be disposed on a left lower end of the space between the face and the oral cavity of the patient.

In an embodiment, the oral balloon may further include a first fluid bag separation gap formed between the first fluid expansion part and the second fluid expansion part and a second fluid bag separation gap formed between the third fluid expansion part and the fourth fluid expansion part.

In an embodiment, the first fluid bag separation gap may be a membrane connecting the first fluid expansion part and the second fluid expansion part, and the second fluid bag separation gap may be a membrane connecting the third fluid expansion part and the fourth fluid expansion part.

In an embodiment, the oral balloon may further include a first insertion guide part (41) having one end connected to the bridge part and the other end connected to an end of the first fluid expansion part and an end of the second fluid expansion part and a second insertion guide part (42) having one end connected to the bridge part and the other end connected to an end of the third fluid expansion part and an end of the fourth fluid expansion part

In an embodiment, a space for accommodating the first implant (15) that is formed by filling a material made of fluid or a flexible material may be formed in the first insertion guide part and the second insertion guide part.

In an embodiment, a second fluid injection port (16) for injecting fluid into the space for accommodating the first implant may be further provided.

In an embodiment, a pocket having an inlet formed toward a central portion of the oral balloon may be formed in at least one of the first fluid expansion part and the second fluid expansion part and at least one of the third fluid expansion part and the fourth fluid expansion part.

In an embodiment, the fluid injection port may have a tube shape having both ends and a cross-section that is gradually increased in a direction from a first end connected to the fluid bag to a second end that is not connected to the fluid bag.

In an embodiment, the fluid injection port may have a tube shape, and an open end of the fluid injection port may be cut obliquely with respect to an extension direction of the fluid injection port.

In an embodiment, ultrasonic energy or RF wave energy passing through the fluid may be decreased while passing through the fluid.

In other embodiments of the present invention, a method for installing the oral balloon in a space between a face and an oral cavity of a patient includes: arranging the first fluid expansion part at a right upper end in the space between the face and an oral cavity of the patient through a mouth of the patient, the second fluid expansion part at a right lower end in the space through the mouth, the third fluid expansion part at a left upper end in the space through the mouth, and the fourth fluid expansion part at a left lower end in the space through the mouth; expanding the fluid bag by injecting fluid into the fluid injection port; and bending a portion of the fluid injection port to prevent the fluid from flowing out of the fluid bag.

In other embodiments of the present invention, a method for installing the oral balloon in a space between a face and an oral cavity of a patient includes: inserting the first insertion guide part into a right side of the space through a mouth of the patient and the second insertion guide part into a left side of the space through the mouth of the patient; and expanding the fluid bag by injecting fluid into the fluid injection port.

In other embodiments of the present invention, a method for installing the oral balloon in a space between a face and an oral cavity of a patient includes: inserting the first insertion guide part into a right side of the space through the mouth of the patient and the second insertion guide part into a left side of the space through the mouth; injecting fluid into the second fluid injection port to expand a space for accommodating the first implant; and expanding the fluid bag by injecting fluid into the fluid injection port.

In other embodiments of the present invention, a method for installing the oral balloon in a space between a face and an oral cavity of a patient includes: expanding the fluid bag by injecting fluid into the fluid injection port; arranging the first fluid expansion part at a right upper end in the space through a mouth of the patient, the second fluid expansion part at a right lower end in the space through the mouth, the third fluid expansion part at a left upper end in the space through the mouth, and the fourth fluid expansion part at a left lower end in the space through the mouth; and bending a portion of the fluid injection port to prevent the fluid from flowing out of the fluid bag.

In other embodiments of the present invention, an oral balloon includes an eleventh fluid expansion part (111), a twelfth fluid expansion part (112), a fluid bag (113) formed in each of the eleventh fluid expansion part (111) and the twelfth fluid expansion part (112), and a fluid injection port (14) for injecting fluid into the fluid bag.

In an embodiment, the oral balloon may further include a bridge part (19) connecting the eleventh fluid expansion part and the twelfth fluid expansion part. Here, the fluid bag may have an integrated shape formed over the eleventh fluid expansion part, the twelfth fluid expansion part, and the bridge part, and the fluid injection port may be formed in the bridge part.

In an embodiment, a pocket having an inlet formed toward a central portion of the oral balloon may be formed in the eleventh fluid expansion part and the twelfth fluid expansion part.

In other embodiments of the present invention, a method for installing the oral balloon in a space between a face and an oral cavity of a patient includes: arranging the eleventh fluid expansion part at a right side in the space through a mouth of the patient and the twelfth fluid expansion part at a left side in the space through the mouth; expanding the fluid bag by injecting fluid into the fluid injection port; and bending a portion of the fluid injection port to prevent the fluid from flowing out of the fluid bag.

In other embodiments of the present invention, a method for installing the oral balloon in a space between a face and an oral cavity of a patient includes: expanding the fluid bag by injecting fluid into the fluid injection port; arranging the eleventh fluid expansion part at a right side in the space through a mouth of the patient and the twelfth fluid expansion part at a left side in the space through the mouth; and bending a portion of the fluid injection port to prevent the fluid from flowing out of the fluid bag.

### ADVANTAGEOUS EFFECTS

Due to the structural features described above, the present invention may include the energy transfer characteristic control tube of the ultrasonic wave and the RF wave, which may be disposed in the oral cavity of the treatment subject (patient), to prevent the energy of the ultrasonic wave and the RF wave from being transmitted to bone tissues such as teeth, gums, and cheekbones.

### BRIEF DESCRIPTION OF THE DRAWINGS

FIG 1 is a plan view of an energy transfer control device according to an embodiment of the present invention.
FIG 2 is a view illustrating a relative position of a face of a patient and the energy transfer control device when the energy transfer control device in FIG 1 is inserted into an oral cavity.
FIGS. 3a to 3d are views for explaining an overall configuration of an energy transfer control device and a purpose of introducing a fluid bag separation gap according to different embodiments.
FIG 4 is a view illustrating a structure of an energy transfer control device according to a modified embodiment of the present invention.
FIG 5 is a view illustrating a structure of an energy transfer control device according to another modified embodiment of the present invention.
FIGS. 6 to 9 are views illustrating products according to various ideas conceived to complete the present invention.
FIG 10 is a view illustrating an energy transfer control device in which the pocket 70 is formed according to an embodiment of the present invention.
FIG 11 is a view illustrating images obtained by designing the shape the energy transfer control device 1 according to an embodiment of the present invention.
FIG 12 is a view illustrating an energy transfer control device according to an embodiment of the present invention.
FIG 13 is a cross-sectional view taken along line H-H in the energy transfer control device 1 of FIG 12.
FIG 14 is a view for explaining an optimized shape of an open end of the fluid injection port 14 according to an embodiment of the present invention.
FIG 15 is a front view of an oral balloon according to another embodiment of the present invention.
FIG 16 is a flowchart for explaining a method for manufacturing an oral balloon according to an embodiment of the present invention.
FIG 17 is a view illustrating a first sheet and a second sheet of the oral balloon according to an embodiment of the present invention.
FIG 18a is a front view of the oral balloon in a state in which the second chamber is not filled with fluid according to an embodiment of the present invention, and FIG 18b is a front view of the oral balloon in a state in which the second chamber is filled with the fluid according to an embodiment of the present invention.
FIG 19 is a front view illustrating the first and second chambers of the oral balloon in the state in which the second chamber filled with the fluid according to an embodiment of the present invention.

### MODE FOR CARRYING OUT THE INVENTION

Hereinafter, embodiments of the present invention will be described in detail with reference to the accompanying drawings. Note that the same or similar components in the drawings are designated by the same reference numerals as far as possible even if they are shown in different drawings. In the following description of the present disclosure, a detailed description of known functions and configurations incorporated herein will be omitted to avoid making the subject matter of the present disclosure unclear.

In the description of the elements of the present disclosure, the terms 'first', 'second', 'A', "B", '(a)', and "(b)" may be used. However, since the terms are used only to distinguish an element from another, the essence, sequence, and order of the elements are not limited by them. When it is described that an element is "coupled to", "engaged with", or "connected to" another element, it should be understood that the element may be directly coupled or connected to the other element but still another element may be "coupled to", "engaged with", or "connected to" the other element between them.

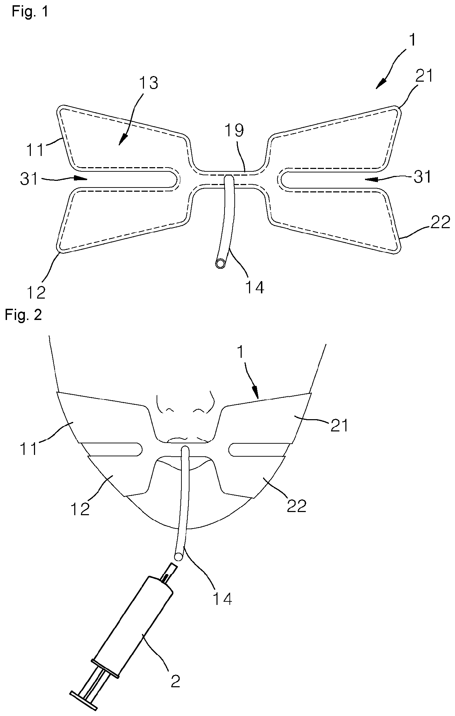

FIG. 1 is a plan view of an energy transfer control device according to an embodiment of the present invention.

FIG. 2 is a view illustrating a relative position of a face of a patient and the energy transfer control device when the energy transfer control device in FIG 1 is inserted into an oral cavity. FIG. 2 is a view illustrating a state in which a energy transfer control device 1 in FIG 1 is inserted between an outer surface of teeth and lips of the patient and between the outer surface of the teeth and an inner surface of a cheek of the patient.

The energy transfer control device 1 may be also referred to as a shock wave transfer characteristic control tube 1 or an oral balloon 1.

Hereinafter, the present invention will be described with reference to FIGS. 1 and 2.

The energy transfer control device 1 that is a device including four fluid expansion parts 11, 12, 21, and 22, a fluid bag 13, a bridge part 19, and a fluid injection port 14 may include a synthetic resin.

The fluid bag 13 may extend over the fluid expansion parts.

When an embodiment to help understanding is explained, the energy transfer control device 1 may be manufactured by stacking two thin sheets of synthetic resins (e.g., vinyl) and attaching edges thereof to each other. Here, a space between non-attached surfaces in the two sheets of synthetic resins may form the fluid bag 13. A dotted line in FIG 1 represents an outer boundary of the fluid bag 13. However, the embodiment of the present invention is not limited thereto.

The first fluid expansion part 11 may be inserted into a space between an inner surface of a cheek/chin skin tissue and gums of the patient, particularly into an upper right portion of the space. The upper right portion that is described from patient's point of view represents a upper left portion is from operator's point of view.

The second fluid expansion part 12 may be inserted into the space between the inner surface of the cheek/chin skin tissue and the gums of the patient, particularly into a lower right portion of the space. The third fluid expansion part 21 may be inserted into the space between the inner surface of the cheek/chin skin tissue and the gums of the patient, particularly into an upper left portion of the space. The fourth fluid expansion part 22 may be inserted into the space between the inner surface of the cheek/chin skin tissue and the gums of the patient, particularly into a lower left portion of the space.

The fluid bag 13 may have a shape connected over each of the fluid expansion parts 11, 12, 21, and 22.

No fluid may be injected into the fluid bag 13 in a state before inserting the energy transfer control device 1 into the oral cavity.

As illustrated in FIG 2, after the energy transfer control device 1 is inserted into the oral cavity, fluid such as air or liquid may be injected into the fluid bag 13 through the fluid injection port 14. For example, a syringe 2 may be inserted into the fluid inlet 14 to inject fluid. When the fluid is injected into the fluid injection port 14, the fluid expansion parts 11, 12, 21, and 22 may be expanded.

Alternatively, after the fluid is injected to the fluid injection port 14 of the energy transfer control device 1 to expand the fluid bag 13, the energy transfer control device 1 may be inserted into the oral cavity. Here, as illustrated in FIG 2, the first fluid expansion part 11 may be disposed on an upper right end of a space of the oral cavity through a mouth of the patient, the second fluid expansion part 12 may be disposed on a lower right end of the space through the mouth, the third fluid expansion part 21 may be disposed on an upper left end of the space through the mouth, and the fourth fluid expansion part 22 may be disposed on a lower left end of the space through the mouth. Here, a portion of the fluid injection port 14 may be bent to prevent the fluid from flowing out of the fluid bag 13.

In an embodiment, the expanded fluid expansion parts 11, 12, 21, and 22 may have a shape disposed over a facial skin area to which an ultrasonic treatment or a RF wave treatment using a separate medical device (not shown) is applied.

Since an ultrasonic wave that reaches the fluid injected into the fluid expansion parts 11, 12, 21, and 22 transfers energy thereof to the fluid during the ultrasonic treatment, ultrasonic energy that reaches the gums and bone tissues of the patient, which are disposed at an inner side of the energy transfer control device 1 decreases.

In this case, e.g., the fluid may be heated by the ultrasonic energy. The fluid may be, e.g., air or liquid.

The bridge part 19 is a portion to which the fluid injected through the fluid injection port 14 firstly reaches, and a portion of the fluid bag 13 is also provided in the bridge part 19. The bridge part 19 may provide a function of connecting the first fluid expansion part 11, the second fluid expansion part 12, the third fluid expansion part 21, and the fourth fluid expansion part 22 to each other.

Although the bridge part 19 may have a significant volume, the bridge part 19 may have substantially no volume. That is, in an embodiment, the bridge part 19 may be merely a conceptual component to distinguish the first fluid expansion part 11, the second fluid expansion part 12, the third fluid expansion part 21, and the fourth fluid expansion part 22 from each other as a point at which the first fluid expansion part 11, the second fluid expansion part 12, the third fluid expansion part 21, and the fourth fluid expansion part 22 are coupled to each other.

However, when the fluid injection port 14 that will be described later is defined in the bridge part 19, the bridge part 19 may have a minimum volume for defining the fluid injection port 14.

The fluid injection port 14 may be defined at one point of the bridge part 19. For example, the fluid injection port 14 may have a flexible tube shape having a predetermined length. An instrument for fluid injection, e.g., a needle of the syringe 2, may be mounted to an end of the fluid injection port 14. After injection of the fluid is completed, the fluid injection port 14 may be simply bound or bent to prevent the injected fluid from flowing out again.

In another embodiment, the fluid injection port 14 may be formed in another portion instead of the bridge part 19, e.g., one of the first fluid expansion part 11, the second fluid expansion part 12, the third fluid expansion part 21, and the fourth fluid expansion parts 22.

After inserting the energy transfer control device 1 into the oral cavity, only the fluid injection port 14 may be exposed outside the lips when the patient closes the lips.

As illustrated in FIG 2, the bridge part 19 may be disposed between an inner surface of the lips and an outer surface of front teeth of the patient. In case that the above-described ultrasonic treatment is performed on the cheek/chin and surrounding facial skin instead of the lips, when a portion of the fluid bag 13 existing at the inside of the lips to which the ultrasonic wave does not reach is excessively expanded, unnecessary discomfort occurs on the lips of the patient. Thus, the fluid bag 13 disposed in the bridge part 19 may be designed to have a minimum volume.

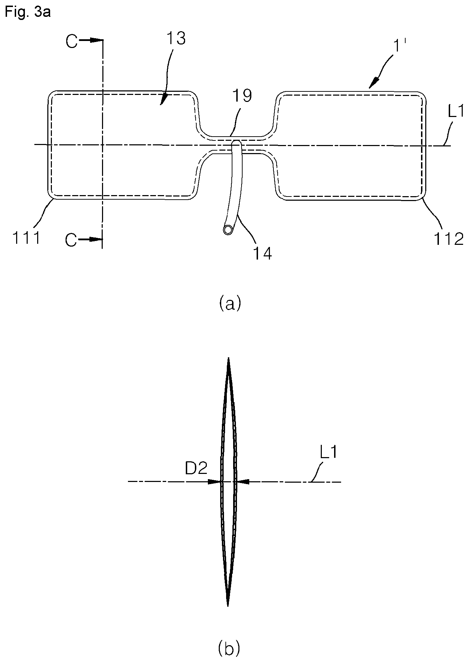

FIGS. 3a to 3d are views for explaining an overall configuration of an energy transfer control device and a purpose of introducing a fluid bag separation gap 31 according to different embodiments.

FIG. 3a shows, unlike FIG 1, a shape in which the fluid expansion part inserted into the oral cavity of the right cheek/chin of the patient and the fluid expansion part inserted into the oral cavity of the left cheek/chin of the patient are integrated into one body instead of being separated into two upper and lower parts according to another embodiment of the present invention.

(a) of FIG 3a is a plan view of a state in which fluid is injected into the fluid bag 13, and (b) of FIG 3a is a cross-sectional view taken along line C-C in (a) of FIG 3a.

An energy transfer control device 1' may include an eleventh fluid expansion parts 111, a twelfth fluid expansion part 112, a fluid bag 113, a fluid injection port 14, and a bridge part 19.

The fluid bag 113 may be formed in the eleventh fluid expansion part 111 and the twelfth fluid expansion part 112.

The fluid injection port 14 may be formed to inject fluid into the fluid bag 113.

The bridge part 19 may connect the eleventh fluid expansion part 111 to the twelfth fluid expansion part 112.

The fluid bag 113 may have an integrated shape over the eleventh fluid expansion part 111, the twelfth fluid expansion part 112, and the bridge part 19.

Here, the fluid injection port 14 may be formed in the bridge part 19.

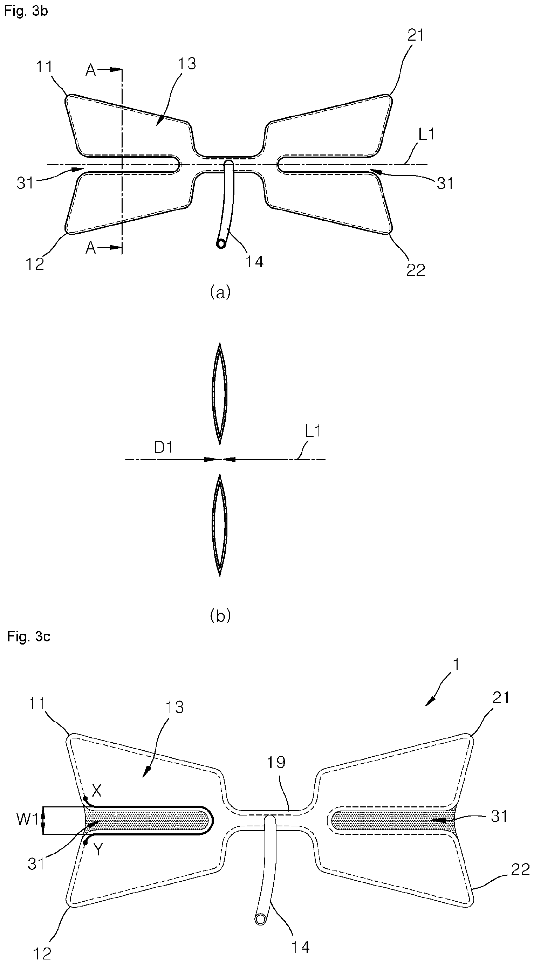

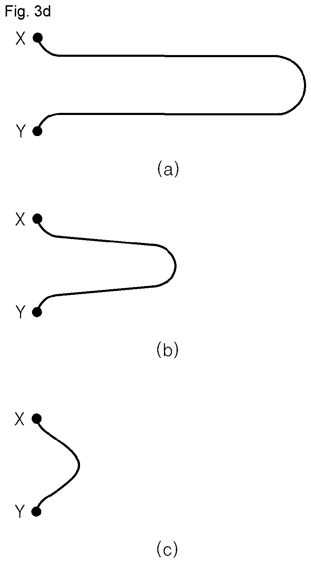

As described above in FIG 2, a method for installing the energy transfer control device 1' of (a) of FIG 3 in a space between a face of the oral cavity of the patient may be performed in a following order.

First, the eleventh fluid expansion part 111 may be arranged at a right side of the space through a mouth of the patient, and the twelfth fluid expansion part 112 may be arranged at a left side of the space through the mouth. Thereafter, fluid may be injected into the fluid injection port 14 to expand the fluid bag 113. Finally, a portion of the fluid injection port 14 may be bent to prevent the fluid from flowing out of the fluid bag 113.

Alternatively, the method for installing the energy transfer control device 1' of (a) of FIG 3 in the space between the face of the oral cavity of the patient may be performed in a following order.

First, fluid may be injected into the fluid injection port 14 to expand the fluid bag 113. Thereafter, the eleventh fluid expansion part 111 may be arranged at the right side of the space through the mouth of the patient, and the twelfth fluid expansion part 112 may be arranged at the left side of the space through the mouth. Finally, a portion of the fluid injection port 14 may be bent to prevent the fluid from flowing out of the fluid bag 113.

FIG 3b shows that, as with FIG 1, a shape in which the fluid expansion part inserted into the oral cavity of the right cheek/chin of the patient is separated into two upper and lower parts, and the fluid bag separation gap 31 inserted into the oral cavity of the left cheek/chin of the patient is separated into two upper and lower parts.

(a) of FIG 3b is a plan view of a state in which fluid is injected into the fluid bag 13, and (b) of FIG 3b is a cross-sectional view taken along line A-A in (a) of FIG 3b.

Unlike the energy transfer control device 1' of FIG 3a, an energy transfer control device 1 of FIG 3b has a fluid bag separation gap 31 that is an empty space. Although the fluid bag 13 is formed without the fluid bag separation gap as illustrated in FIG 3a, the fluid bag 13 may help to prevent pain in the oral cavity of the patient. However, when a fluid bag separation gap is added as illustrated in FIG 3b, the effect may further increase.

Hereinafter, a reason why this fluid bag separation gap 31 is more effective and a purpose of introduction thereof will be described with reference to FIGS. 3a and 3b.

When viewed based on a horizontal central line L1, in case of the energy transfer control device 1 illustrated in FIG 3a, a thickness D2 of the fluid bag at the horizontal central line L1 increases in a state in which the fluid is injected. Here, a portion at the horizontal central line L1 may put a pressure on a treatment area of the patient, and some patients may feel the pressure uncomfortable.

In comparison with this, when viewed based on the horizontal central line L1, in case of the energy transfer control device 1 illustrated in FIG 3b, a thickness D1 of the fluid bag at the horizontal central line L1 in a state in which the fluid is injected is extremely thin. Thus, the above-described problem in which the portion at the horizontal central line L1 puts the pressure on the treatment area of the patient is solved. Also, as the fluid bag separation gap 31 is introduced, a maximum thickness of each of the first fluid expansion part 11, the second fluid expansion part 12, the third fluid expansion part 21, the fourth fluid expansion part 22 is naturally reduced, and the discomfort of the patient is reduced although the energy transfer control device 1 is inserted into the oral cavity to expand the fluid bag.

Referring to FIGS. 1 and 3c, a fluid bag separation gap 31 is disposed between a first fluid expansion part 11 and a second fluid expansion part 12.Also, the fluid bag separation gap 31 is disposed between a third fluid expansion part 21 and a fourth fluid expansion part 22. In the example of FIG 1, the fluid bag separation gap 31 represents a space between the first fluid expansion part 11 and the second fluid expansion part 12. In the example of FIG 3c, the fluid bag separation gap 31 represents a membrane formed between the first fluid expansion part 11 and the second fluid expansion part 12. The membrane may be also made of the synthetic resin.

As described above, it may be understood that a predetermined technical effect is achieved by the introduction of the fluid bag separation gap 31 in FIGS. 1 and 3c.

Referring to FIG 3c again, a vertical width generated as the first fluid expansion part 11 and the second fluid expansion part 12 are separated from each other by the fluid bag separation gap 31 is expressed by W1.Since fluid is not provided to the vertical width W1, ultrasonic energy may directly reach the gums and bone tissue of the patient when the ultrasonic treatment is performed on the skin on the fluid bag separation gap 31. Thus, it may be desirable to minimize the vertical width W1.

On the other hand, referring to FIG 3c, a curved line X-Y may be defined along an edge of the fluid bag 31 formed on the first fluid expansion part 11 and the second fluid expansion part 12. The curved line X-Y has a concave shape from a left edge to a center of the energy transfer control device 1. The curved line X-Y has a structural feature provided to achieve an improved effect of the present invention described through FIGS. 3a and 3b.

As illustrated in FIG 3d, although the curved line X-Y may be designed to have various curvatures and shapes, a component corresponding to the above-described fluid bag separation gap 31 is provided regardless of a shape, and the component corresponding to the fluid bag separation gap 31 exhibits an effect of preventing excessive expansion of the fluid expansion part. Thus, this may be considered to fall within the scope of the present invention.

Also, according to the embodiment in FIG 1, although two distinguished first fluid expansion parts 11 and second fluid expansion parts 12 are provided on a left side of the bridge part 19, the additional third fluid expansion part may be provided at the left side. Here, a separation gap structure corresponding to the above-described fluid bag separation gap 31 may be further provided between the third fluid expansion part and the first fluid expansion part 11 or the second fluid expansion part 12. That is, a configuration in which two or three or more fluid expansion parts that are disposed at the left or right side based on the bridge part 19 and distinguished from each other in the vertical direction is included in the concept of the present invention.

In an embodiment, the third fluid expansion part 21 and the fourth fluid expansion part 22 may be symmetric to the first fluid expansion part 11 and the second fluid expansion part 12, respectively. Thus, the separation gap 31 between the third fluid expansion part 21 and the fourth fluid expansion part 22 may have the same structure as the separation gap 31 including the above-described curved line X-Y with respect to the first fluid expansion part 11 and the second fluid expansion part 12.

Although a left and right width of the bridge part 19 is not 0 (zero) in FIG 1, the embodiment of the present invention is not limited thereto. Since the bridge part 19 that is a part in which the fluid injection port 14 is formed or connected has a substantial volume, the term is introduced to express this.

Although the fluid injection port 14 is illustrated as a long pipe in FIG 1, an embodiment in which the length of the pipe is close to zero is also included in the concept of the present invention. When the length of the pipe is 0, the fluid injection port 14 may simply exist as an opening.

Also, although one fluid injection port 14 is formed at a central portion of the energy transfer control device 1 in FIG 1, two or more fluid injection ports 14 may be installed at any position in which fluid is injectable into the fluid bag 13 depending on embodiments. All of these modified embodiments are included in the present invention.

In a modified embodiment of the present invention, the first fluid expansion part 11, the second fluid expansion part 12, the third fluid expansion part 21, and the fourth fluid expansion part 22 may not be connected to each other by one fluid bag 13. That is, for example, empty spaces of the first fluid expansion part 11 and the second fluid expansion part 12 may be connected to each other by one first fluid bag 13_1, and empty spaces of the third fluid expansion part 21 and the fourth fluid expansion unit 22 may be connected to each other by one second fluid bag 13_2. Here, the first fluid bag 13_1 and the second fluid bag 13_2 may be separated from each other. Here, the fluid injection port 14 may be installed separately in the first fluid bag 13_1 and the second fluid bag 13_2.

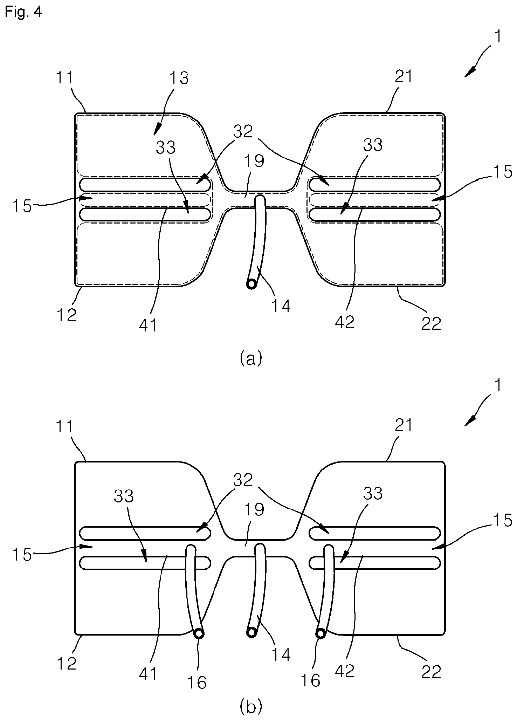

FIG 4 is a view illustrating a structure of an energy transfer control device 1 according to a modified embodiment of the present invention.

The energy transfer control device 1 illustrated in FIG 4 basically has the structure of the energy transfer control device 1 illustrated in FIG 1, and an additional component is added thereto. Hereinafter, when FIG 4 is described, a description that overlaps that in FIG 1 may be omitted.

Since the energy transfer control device 1 is basically in an extremely flexible state before injecting fluid, a lot of efforts are required to set the fluid expansion parts 11, 12, 21, and 22 at necessary positions after inserted into the oral cavity of the patient. That is, since an operator holds the extremely flexible fluid expansion parts and move the same in the mouth of the patient, a lot of time and efforts are required, and the patient may feel great discomfort. FIG 4 shows the additional component according to an embodiment of the present invention to solve the above-described problem.

(a) of FIG 4 is a view illustrating a case in which a fluid injection port 14 is provided only to a bridge part 19 of an energy transfer control device 1, and (b) of FIG 4 is a view illustrating a case in which a second fluid injection port 16 is provided to each the insertion guide part 41 and the second insertion guide part 42 of an energy transfer control device 1.

First, the case of (a) of FIG 4 will be described.

The energy transfer control device 1 may further include a first insertion guide part 41 and a second insertion guide part 42. Each of the first insertion guide part 41 and the second insertion guide part 42 may be additionally disposed at the position of the above-described fluid bag separation gap 31.

The first insertion guide part 41 has one end connected to the bridge part 19 and the other end connected to the first fluid expansion part 11 and a right end of the second fluid expansion part 12 (when viewed from a patient's perspective).

A second fluid bag separation gap 32 may be formed between the first insertion guide part 41 and the first fluid expansion part 11, and a third fluid bag separation gap 33 may be formed between the first insertion guide part 41 and the second fluid expansion part 12. The second fluid bag separation gap 32 may be an empty space or a thin-film connected to the first fluid expansion part 11 and the first insertion guide part 41. The third fluid bag separation gap 33 may be an empty space or a thin-film connected to the second fluid expansion part 12 and the first insertion guide part 41.

Each of the second fluid bag separation gap 32 and the third fluid bag separation gap 33 may be a term that refers to a feature in which the fluid bag separation gap 31 is separated by the first insertion guide part 41. Thus, the second fluid bag separation gap 32 and the third fluid bag separation gap 33 may be collectively referred to as the fluid bag separation gap 31.

A first implant 15 for easily inserting the energy transfer control device 1 into the oral cavity may be formed in the first insertion guide part 41.

The first implant 15 may be fluid. Here, the fluid may allow the first insertion guide part 41 to be tightly expanded from the inside to the outside thereof, so that the first insertion guide part 41 may maintain a shape despite of a force applied to the first insertion guide part 41. The fluid may be inserted in advance in a process of manufacturing the energy transfer control device 1. Alternatively, the fluid may be inserted during a procedure as described in (b) of FIG 4.

Alternatively, the first implant 15 may be, instead of the fluid, a synthetic resin having elasticity while maintaining a shape or a synthetic resin combined with metal.

When the first implant 15 is the fluid, a space occupied by the first implant 15 is separated from the fluid bag 13 instead of being connected thereto.

Since the second insertion guide part 42 also has the same configuration as the first insertion guide part 41, a detailed description thereof will be omitted.

(b) of FIG 4 is a modified embodiment from (a) of FIG 4.

When the first implant 15 is the fluid, the first implant 15 may be formed by the operator during a use process instead of being inserted in the process of manufacturing the energy transfer control device 1. To this end, the second fluid injection port 16 may be provided in each of the first insertion guide part 41 and the second insertion guide part 42.

The second fluid injection port 16 is connected to the first implant 15, i.e., a space into which the fluid is injected (i.e., the first insertion guide part 41 and the second insertion guide part 42). The space is separated from the above-described fluid bag 13.

The fluid may be injected firstly through the second fluid injection port 16 before the operator inserts the energy transfer control device 1 into the oral cavity of the fluid.

Thereafter, the operator may push the first insertion guide part 41 into the right oral cavity of the patient and the second insertion guide part 42 into the left oral cavity of the patient to insert the energy transfer control device 1 into the oral cavity of the patient. Here, the fluid expansion parts 11 and 12 in the extremely flexible state may be inserted into the first insertion guide part 41, and the fluid expansion parts 21 and 22 in the extremely flexible state may be inserted into the second insertion guide part 42.

Thereafter, the operator may expand the fluid expansion parts 11, 12, 21, and 22 by injecting the fluid into the fluid bag 13 through the fluid injection port 14.

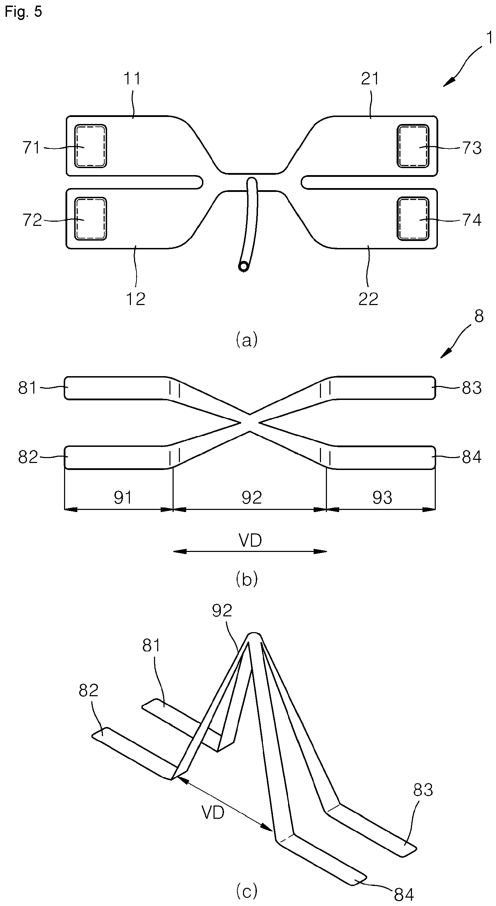

FIG 5 is a view illustrating a structure of an energy transfer control device 1 according to another modified embodiment of the present invention.

The energy transfer control device 1 illustrated in FIG 5 basically has the structure of the energy transfer control device 1 illustrated in FIG 1, and an additional component is added thereto. Hereinafter, when FIG 4 is described, a description that overlaps that in FIG 1 may be omitted.

(a) of FIG 5 is a view illustrating an energy transfer control device 1 in which pockets are formed in fluid expansion parts.

As illustrated in (a) of FIG 5, the energy transfer control device 1 may include fluid expansion parts 11, 12, 21, and 22.Pockets 71, 72, 73, and 74 may be formed in the fluid expansion parts 11, 12, 21, and 22, respectively. The pocket has a shape similar to a pants pocket, and an insertion port of the pocket is formed toward a central portion of the energy transfer control device 1.

(b) of FIG 5 is a view illustrating forceps having spatula parts 81, 82, 83, and 84 that are insertable into the pockets 71, 72, 73, and 74, respectively.

The forceps 8 may include a left spatula part 91, a right spatula part 93, and a handle 92.

The left spatula part 91 may include a first left spatula part 81 and a second left spatula part 82. The right spatula part 93 may include a first right spatula part 83 and a second right spatula part 84.

The spatula parts 81, 82, 83, and 84 may be inserted into the pockets 71, 72, 73, and 74, respectively. That is, the first left spatula part 81 may be inserted into the first pocket 71, the second left spatula part 82 may be inserted into the second pocket 72, the first right spatula part 83 may be inserted into the third pocket 73, and the second right spatula part 84 may be inserted into the fourth pocket 74.

The forceps 8 may be referred to as an insertion instrument 8.

(c) of FIG 5 is a perspective view of the forceps 8 illustrated in (b) of FIG 5.

A left and right lengths VD of the handle 92 (i.e., a length between a first point that is a start point of the left spatula part 91 and an end point of the handle 92 and a second point that is a start point of the right spatula part 93 and an end point of the handle 92) may be adjusted by a force held by a hand of the operator.

Hereinafter, a preparation process for the operator to insert the energy transfer control device 1 into the oral cavity of the patient and for use of the energy transfer control device 1 will be described.

First, the spatula parts 81, 82, 83, and 84 may be inserted into the pocket 71, 72, 73, and 74, respectively, before the operator inserts the energy transfer control device 1 into the oral cavity of the patient.

Thereafter, the operator may insert the energy transfer control device 1 into the oral cavity of the patient by applying a force to reduce the left and right length VD of the forceps 8 coupled with the energy transfer control device 1. Thereafter, the operator allow the left and right length VD to be increased by releasing the force applied to the forceps 8 coupled with the energy transfer control device 1. When it is determined that the energy transfer control device 1 is unfolded in the oral cavity of the patient, the operator may apply a force to reduce the left and right length VD of the forceps 8 again, thereby removing the forceps 8 from the oral cavity while deforming a shape of the forceps 8.

Thereafter, the operator may expand the fluid expansion parts 11, 12, 21, and 22 by injecting the fluid into the fluid bag 13 through the fluid injection port 14.

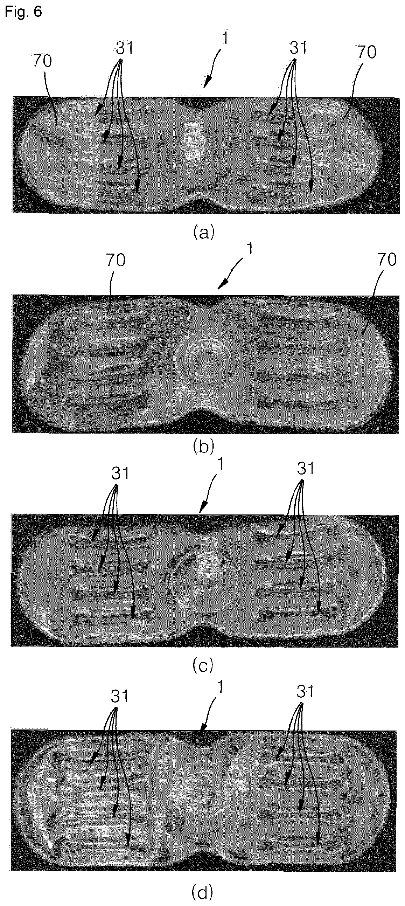

FIGS. 6 to 9 are views illustrating products according to various ideas conceived to complete the present invention.

FIG 6 is a front and back view illustrating a case in which the pocket is formed in the energy transfer control device and a case in which the pocket is not formed in the energy transfer control device according to an embodiment of the present invention.

(a) of FIG 6 is a plan view of the energy transfer control device 1 in which a pocket 70 is formed, (b) of FIG 6 is a rear view of the energy transfer control device 1 in which the pocket 70 is formed, (c) of FIG 6 is a plan view of the energy transfer control device 1 in which the pocket is not formed, and (d) of FIG 6 is a rear view of the energy transfer control device in which the pocket is not formed.

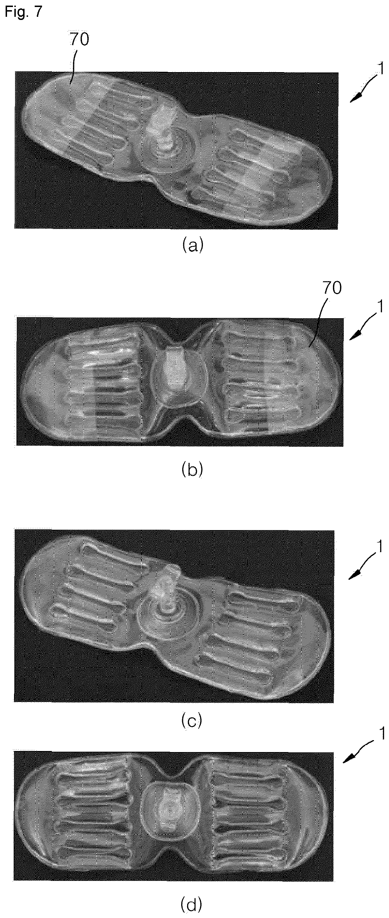

FIG 7 is a view illustrating states before and after the energy transfer control device is expanded in each of the case in which the pocket is formed in the energy transfer control device and the case in which the pocket is not formed in the energy transfer control device according to an embodiment of the present invention.

(a) of FIG 7 is a view illustrating a state before the energy transfer control device 1 in which the pocket 70 is formed is expanded, (b) of FIG 7 is a view illustrating a state after the energy transfer control device 1 in which the pocket 70 is formed is expanded, (c) of FIG 7 is a view illustrating a state before the energy transfer control device 1 in which the pocket 70 is not formed is expanded, and (d) of FIG 7 is a view illustrating a state after the energy transfer control device 1 in which the pocket 70 is not formed is expanded.

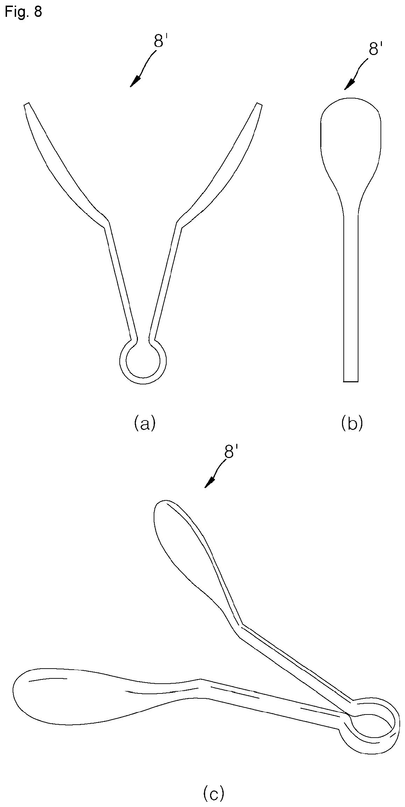

FIG 8 is a view illustrating an appearance of the forceps according to an embodiment of the present invention.

(a) of FIG 8 is a front view of forceps 8', (b) of FIG 8 is a side view of the forceps 8', and (c) of FIG 8 is a perspective view of the forceps 8'.

Although the embodiment in which the forceps 8 have four spatula parts is described in FIG 5, it may be known from FIG 8 that the forceps 8' may have two spatula parts.

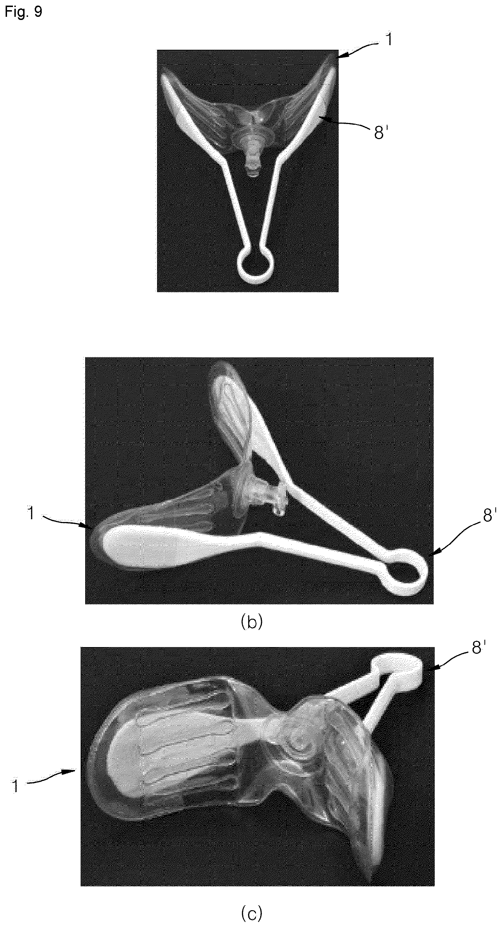

FIG 9 is a view illustrating states, in which the forceps are inserted into the energy transfer control device, viewed from various angles according to an embodiment of the present invention.

In the energy transfer control device 1 in FIGS. 6, 7, and 9, a plurality of fluid bag separation gaps 31 are provided in each of the left fluid expansion part and the right fluid expansion part.

Products that are different type of energy transfer control devices illustrated in FIGS. 6 to 9 may have an advantage and a disadvantage according to functions thereof.

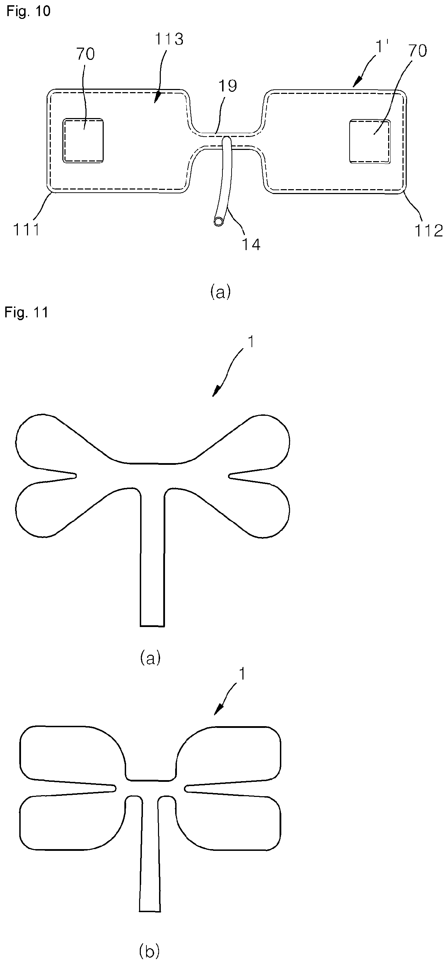

FIG 10 is a view illustrating the energy transfer control device 1' in which the pocket 70 is formed according to an embodiment of the present invention.

The pocket 70 having in inlet formed toward a central portion of the energy transfer control device 1' may be formed in each of the eleventh fluid expansion part 111 and the twelfth fluid expansion part 112 of the energy transfer control device 1'.

FIG 11 is a view illustrating images obtained by designing the shape the energy transfer control device 1 according to an embodiment of the present invention.

As illustrated in FIG 11, each of the fluid expansion part and the fluid injection port of the energy transfer control device may have various shapes.

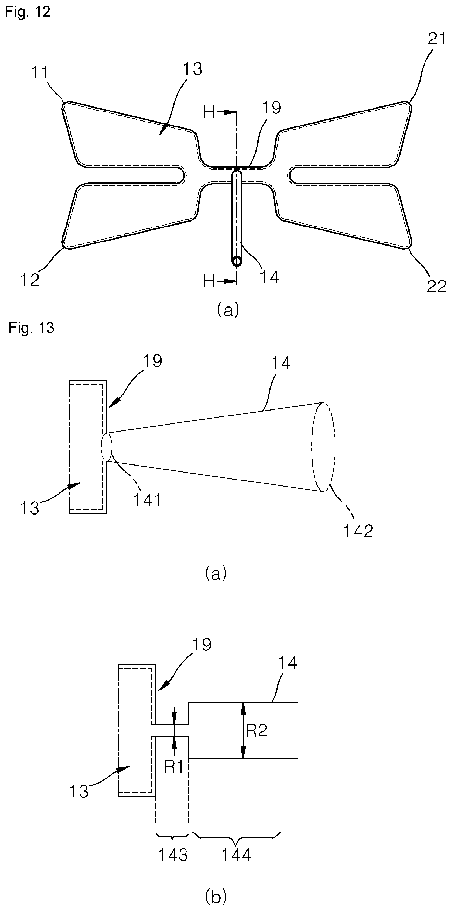

FIG 12 is a view illustrating an energy transfer control device 1 according to an embodiment of the present invention.

As described above, the fluid injected through the fluid injection port 14 reaches the bridge part 19. Since a portion of the fluid bag 13 is also provided to the bridge part 19, the fluid passing through the bridge part 19 is moved to the fluid expansion parts 11, 12, 21, and 22.

FIG 13 is a cross-sectional view taken along line H-H in the energy transfer control device 1 of FIG 12.

(a) of FIG 13 is a view illustrating a fluid injection port 14 according to an embodiment of the present invention, and (b) of FIG 13 is a view illustrating a fluid injection port 14 according to another embodiment of the present invention.

(a) of FIG 13 and (b) of FIG 13 are views for explaining the fluid inlet 14 formed to be easily bent when the fluid injection port 14 is bent to prevent the fluid from leaking out of the fluid injection port 14 after the fluid is injected through the fluid injection port 14.

Hereinafter, a cross-sectional shape of the fluid injection port 14 will be described with reference to FIGS. 12 to 13.

The fluid injection port 14 may have a tube shape having both ends. Here, as illustrated in (a) of FIG 13, a cross-section may be gradually increased in a direction from a first end 141 connected to the fluid bag 13 of the bridge part 19 to a second end 142 that is not connected to the fluid bag among both ends of the fluid inlet 14. Here, since the first end 141 connected to the fluid bag 13 of the bridge part 19 has a narrow cross-section, the fluid injection port 14 in a direction toward the first end 141 (14) may be easily bent after the fluid is injected through the fluid injection port 14.

Alternatively, as illustrated in (b) of FIG 13, the fluid injection port 14 may be divided into a first injection part 143 and a second injection part 144.

The first injection part 143 may have one end connected to the fluid bag 13 of the bridge part 19 and the other end connected to one end of the second injection part 144. Here, the fluid may be firstly injected to the other end of the second injection part 144.

The first injection part 143 may have a diameter R1 less than a diameter R2 of the second injection part 144. That is, a stepped portion may be formed between the first injection part 143 and the second injection part 144. Due to this, since the first injection part 143 may be easily bent, the fluid may not be leaked to the fluid injection port 14 by bending a portion of the first injection part 143 after the fluid is injected through the fluid injection port 14.

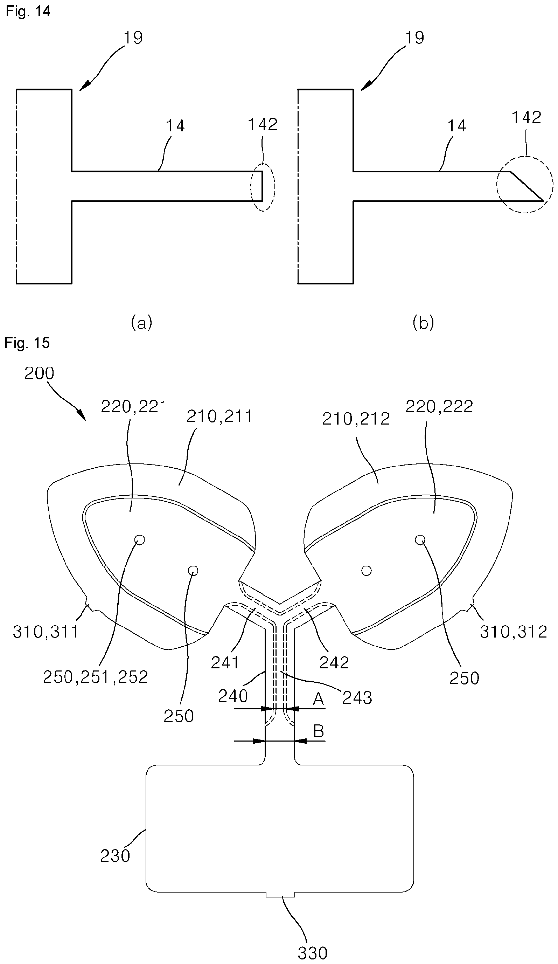

FIG 14 is a view for explaining an optimized shape of an open end of the fluid injection port 14 according to an embodiment of the present invention.

(a) of FIG 14 shows a state in which an open end 142 of the fluid injection port 14 is cut to be parallel to the bridge part 19, and (b) of FIG 14 shows a state in which the open end 142 of the fluid injection port 14 is cut obliquely with respect to an extension direction of the fluid injection port 14.

When the open end 142 of the fluid injection port 14 is cut obliquely as illustrated in (b) of FIG 14 rather than (a) of FIG 14, it may be understood that a fluid injection instrument such as a syringe is easily inserted into the open end 142.

Here, although the fluid injection port 14 having a short length is illustrated for convenience of description in FIGS. 12 to 14, the fluid injection port 14 may have a long length so that a passage of the fluid injection port 14 is automatically blocked by own weight of the fluid injection port 14 when the fluid injection port 14 is bent after the fluid is injected.

FIG 15 is a front view of an oral balloon according to another embodiment of the present invention.

An oral balloon 200 may include a first chamber 210, a second chamber 220, a third chamber 230, and a fluid passage 240. In this specification, a term 'chamber' may be also referred to as 'room' or 'space'. Accordingly, the first chamber, second chamber, and third chamber may be referred to as the first room, the second room, and third room, respectively. Alternatively, the first chamber, second chamber, and third chamber may be referred to as the first room, the second room, and third room, respectively.

The first chamber 210 and the second chamber 220 may be inserted into a space between teeth and an inner wall of lips (and cheeks) of a person.

The first chamber 210 and the second chamber 220 may for one set, and the one set may be disposed at each of both cheeks of the person. For example, one set including a first chamber 211 and a second chamber 221 may be disposed in a space between left teeth and an inner wall of a left cheek of the person, and another set including a first chamber 212 and a second chamber 222 may be disposed in a space between right teeth and an inner wall of a right cheek of the person.

Here, the second chamber 220 may be referred to as a fluid bag in this specification. Also, for example, the second chamber 221 on the left may be referred to as a eleventh fluid expansion part, and the second chamber 222 on the right may be referred to as the twelfth fluid expansion part. That is, the second chamber 220 may be formed by the eleventh fluid expansion part and the twelfth fluid expansion part.

The first chamber 210 may be arranged to surround a portion of an edge of the second chamber 220. Here, the first chamber 210 and the second chamber 220 may be completely separated.

Here, the first chamber 210 may be filled with fluid. The fluid may be referred to as an implant in this specification. The implant may substantially maintain a shape when the oral balloon 200 is inserted into the oral cavity. Here, the implant may include one or more of fluid, a synthetic resin, and metal.

The fluid filled in the first chamber 210 may not be moved to the second chamber 220 because the first chamber 210 is separated from the second chamber 220. Here, the second chamber 220 may not be filled with fluid.

The oral balloon 200 may be manufactured in a state in which the first chamber 210 is filled with fluid in advance, which allows the oral balloon 200 to be easily inserted into the oral cavity. The first chamber 210 may be referred to as an `insertion guide part' in this specification.

A fluid movement passage 240 for receiving fluid from the third chamber 230 may be formed in a portion of an edge of the second chamber 220, which is not surrounded by the first chamber 210.

Hereinafter, the third chamber 230 will be described first before explaining the fluid movement passage 240.

The first chamber 210 and the third chamber 230 may be completely separated.

Here, the third chamber 230 may be filled with fluid as with the first chamber 210. That is, when the oral balloon 200 is manufactured, the third chamber 230 may be manufactured in a state of being filled with fluid.

The second chamber 220 and the third chamber 230 may be connected to each other through the fluid movement passage 240 as described above.

Thus, the first chamber 210 and the third chamber 230 may be connected only through the fluid movement passage 240 and the second chamber 220.

The fluid movement passage 240 may include a first fluid movement passage 241 connected to the second chamber 221 on the left, a second fluid movement passage 242 connected to the second chamber 222 on the right, and a third fluid movement passage 243 connected to the third chamber 230.

In this specification, the first fluid movement passage 241 and the second fluid movement passage 242 may be collectively referred to as a 'bridge part'. Also, the first fluid movement passage 241, the second fluid movement passage 242, and the third fluid movement passage 243 may have a 'Y' shape when viewed from the front. The bridge part may connect the eleventh fluid expansion part that is the second chamber 221 on the left and the twelfth fluid expansion part that is the second chamber 222 on the right.

The first fluid movement passage 241, the second fluid movement passage 242, and the third fluid movement passage 243 of the fluid movement passage 240 may have substantially an integrated shape of connecting the second chamber 220 and the third chamber 230. Also, each of the first fluid movement passage 241, the second fluid movement passage 242, and the third fluid movement passage 243, i.e., each passage, may have an inner width A less than a width B of an inlet passage of each of the second chamber 220 and the third chamber 230. This represents that, when pressure is applied to the third room 230, the fluid is moved to the second chamber 220 through the fluid movement passage 240 to expand the second chamber 220, thereby preventing the fluid flowing to the second chamber 220 to be easily moved again to the third chamber 230.

Here, a width B of an inlet passage of the third chamber 230 based on the third chamber 230 may be a width of the entire fluid movement passage 240, and the width may be gradually increased in a direction from the inlet passage to the inner passage (i.e. B>A).

Here, the third fluid movement passage 243 that is substantially a common passage connected to the bridge parts 241 and 242 to inject the fluid into the second chambers 220 may be referred to as a 'fluid injection port' in this specification. That is, the fluid injection port may be formed in the bridge part.

Hereinafter, the second chamber will be described in detail.

A first point 251 of a first surface (e.g., front surface) and a second point 252 of a second surface (e.g., rear surface) of the second chamber 220 may be bonded to each other. When the fluid is injected into the second chamber 220 by bonding the first point 251 and the second point 252, a three-dimensional shape of the second chamber 220 may be determined (restricted). This effect may be the same as that described above with reference to FIGS. 3a and 3b.

One or more bonded areas 250 may be formed.

The bonded area 250 may be referred to as a 'fluid bag separation gap' in this specification. The bonded area 250 may be obtained by bonding surfaces of the oral balloon 200 and made of the same material as the oral balloon 200. For example, the area 250 may be made of a synthetic resin.

Here, a pocket having an inlet formed toward a central portion of the oral balloon (i.e., fluid movement passage) may be formed in the second area 220. For example, the pocket may have a shape similar to that of (a) of FIG 5.

Hereinafter, the fluid injection port of the oral balloon will be described.

The oral balloon 200 may include fluid injection parts 310 and 330 for injecting fluid into the oral balloon 200.

Specifically, the fluid injection parts 311 and 312 may be formed at a point on one edge of the first chambers 211 and 212 to inject the fluid into the first chambers 211 and 212. Also, the fluid injection part 330 may be formed at a point on one edge of the third chamber 230 to inject the fluid into the third chamber 230.

The fluid injection parts 311, 312, and 330 may be closed by thermal bonding after the fluid is injected during manufacturing.

As illustrated in FIG 15, when a pair of first chambers 210 exist, the first chamber on the left may be referred to as a left first chamber, and the first room on the right may be referred to as a right first chamber. Also, when two second chambers 220 exist, the second chamber on the left may be referred to as a left second chamber, and the second chamber on the right may be referred to as a right second chamber.

The fluid movement passage 240 has a structure in which three passages meet at one point. That is, one end of the first passage, one end of the second passage, and one end of the third passage meet at one point and are connected to each other. Also, the other end of the first passage may be connected to the left second chamber, the other end of the second passage may be connected to the right second chamber, and the other end of the third passage may be connected to the third chamber.

The first chamber 210 may have an English 'C' shape or a Korean '⊏' shape to surround an outer edge of the second chamber 220. A portion of the outer edge of the second chamber 220, which is not surrounded by the first chamber 210, may be connected to the fluid movement passage 240. For example, a portion of the outer edge of the left second chamber, which is not surrounded by the first left chamber, may be connected to the other end of the first passage. Also, a portion of the outer edge of the left second chamber, which is not surrounded by the right first chamber, may be connected to the other end of the second passage.

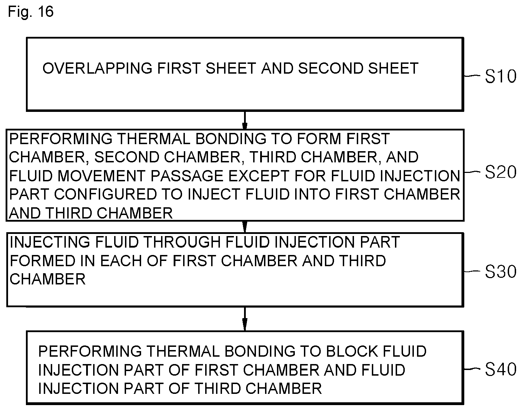

FIG 16 is a flowchart for explaining a method for manufacturing an oral balloon according to an embodiment of the present invention.

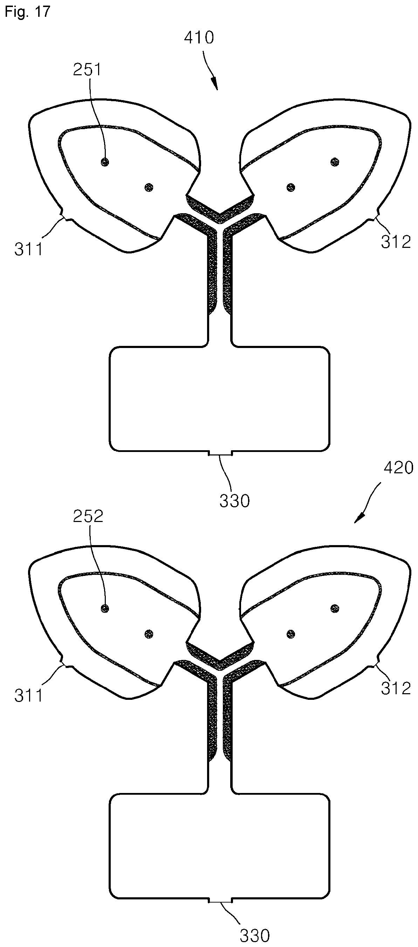

FIG 17 is a view illustrating a first sheet and a second sheet of the oral balloon according to an embodiment of the present invention.

Hereinafter, the present invention will be described with reference to FIGS. 15 and 17.

In step S10, a first sheet 410 and a second sheet 420 may be arranged to overlap each other. Here, the first sheet 410 and the second sheet 420 may be made of a synthetic resin. For example, the first sheet 410 may be arranged on the second sheet 420 in an overlap manner.

In step S20, thermal bonding may be performed to form a first chamber 210, a second chamber 220, a third chamber 230, and a fluid movement passage 240 except for a fluid injection part 310 for injecting fluid into the first chamber 210 and the third chamber 230. Here, a portion to be thermally bonded may be a portion that is colored and expressed by a thick solid line in the first sheet 410 and the second sheet 420 in FIG 17. Here, in the step S20, a first point 251 of the first sheet 410 of the second chamber 220 and a second point 252 of the second sheet 420 of the second chamber 220 may also be thermally bonded. Here, as illustrated in FIG 17, the rest edge (colored portion) except for a first fluid movement passage 241, a second fluid movement passage 242, and a third fluid movement passage 243 among the fluid movement passages 240 may be widely thermally bonded. Alternatively, in another embodiment, only a portion expressed by two dotted lines in the first fluid movement passage 241, the second fluid movement passage 242, and the third fluid movement passage 243 among the fluid movement passages 240 of FIG 15 may be thermally bonded.

In step S30, fluid may be injected through fluid injection parts 311, 312, and 330 formed in the first chamber 210 and the third chamber 230.Here, as illustrated in FIG 17, the fluid injection parts 311, 312, and 330 are expressed by a thin solid line. That is, it may be known that the fluid injection parts 310 and 330 are not thermally bonded until the fluid is injected.

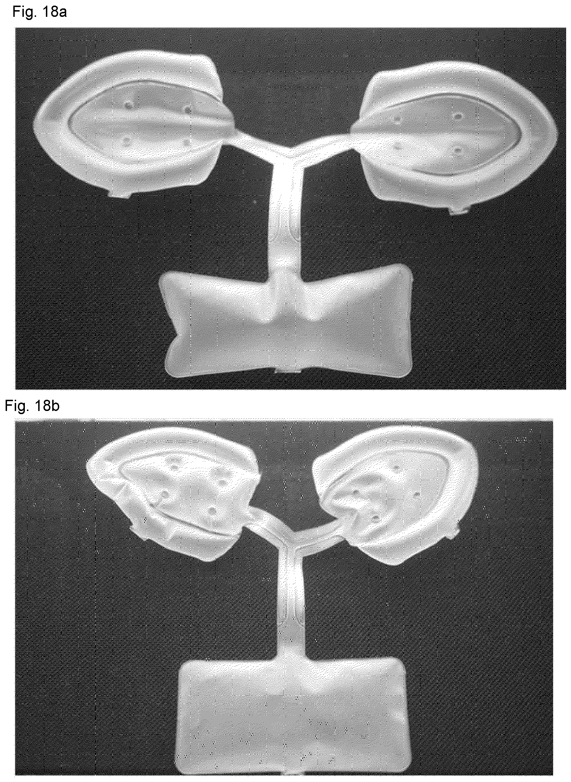

In step S40, the fluid injection part 310 of the first chamber 210 and the fluid injection part 330 of the third chamber 230 may be thermally bonded to be blocked. Here, a state in which the thermal bonding of the oral balloon is completed may be as shown in FIG 18a.

FIG 18a is a front view of the oral balloon in a state in which the second chamber is not filled with fluid according to an embodiment of the present invention, and FIG 18b is a front view of the oral balloon in a state in which the second chamber is filled with the fluid according to an embodiment of the present invention.

Hereinafter, the present invention will be described with reference to FIGS. 15 to 18b.

FIG 18a may be a view of the oral balloon immediately after being manufactured according to the above-described steps in FIG 16. Accordingly, the fluid injection part 310 of the first chamber 210 and the fluid injection part 330 of the third chamber 230 may be blocked through the thermal bonding.

Here, when a user applies pressure to the third chamber 230 of FIG 18a, fluid in the third chamber 230 may be supplied to the second chamber 220 through the fluid movement passage 240, so that the second chamber 220 is expanded. As a result, the expanded second chamber 220 may have an appearance as shown in FIG 18b. Here, since a portion of the fluid in the third chamber 230 is moved, a degree of expansion of the third chamber 230 may be reduced.

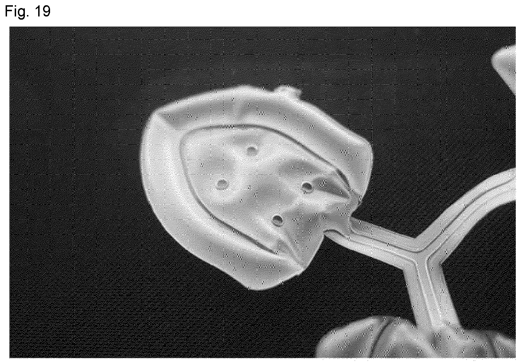

FIG 19 is a front view illustrating the first and second chambers of the oral balloon in the state in which the second chamber filled with the fluid according to an embodiment of the present invention.

The second chamber 220 may be in an expanded state, and, for example, four bonded areas may be formed in the second chamber 220. As the above-described bonded area is formed, the effects described in FIGS. 3a and 3a may be obtained.

Unlike FIG 19, the second chamber 220 may have one bonded area or a plurality of bonded areas.

Hereinafter, a method for installing the above-described oral balloon in a space between a face and an oral cavity of a patient will be described.

First, a set of the first chamber 210 and the second chamber 220 (i.e., fluid bag) may be arranged in a mouth of the patient. For example, one set of the first and second chambers may be arranged at a left side in the mouth of the patient, and another set may be arranged at a right side in the mouth of the patient.

After arranged, pressure may be applied to the third chamber 230 to inject the fluid through the fluid movement passage 240 (i.e., fluid injection port and bridge part), thereby expanding the second chamber 220.

Alternatively, in another embodiment, pressure may be applied to the third chamber 230 to inject the fluid through the fluid movement passage 240 (i.e., fluid injection port and bridge part), thereby expanding the second chamber 220.

Thereafter, the set of the first chamber 210 and the second chamber 220 may be arranged in the mouth of the patient. For example, one set of the first and second chambers may be arranged at the left side in the mouth of the patient, and another set may be arranged at the right side in the mouth of the patient.

The above-disclosed subject matter is to be considered illustrative, and not restrictive, and the appended claims are intended to cover all such modifications, enhancements, and other embodiments, which fall within the true spirit and scope of the present disclosure. Thus, the embodiment of the present invention is to be considered illustrative, and not restrictive, and the technical spirit of the present invention is not limited to the foregoing embodiment. Therefore, the scope of the invention is defined not by the detailed description of the invention but by the appended claims, and all differences within the scope will be construed as being included in the present disclosure.

### [Description of reference numerals]

1: Energy transfer control device
11, 12, 21, 22, 111, 112: Fluid expansion part
13, 113: Fluid bag
14: Fluid injection port
15: First implant
16: Second fluid injection port
19: Bridge part
41: First insertion guide part
42: Second insertion guide part

## Claims

1. An oral balloon comprising:
a fluid bag into which fluid can be injected; and
a fluid injection port configured to inject fluid into the fluid bag.

2. The oral balloon of claim 1, further comprising:
an eleventh fluid expansion part;
a twelfth fluid expansion part; and
a bridge part configured to connect the eleventh fluid expansion part and the twelfth fluid expansion part to each other,
wherein the fluid bag is formed by the eleventh fluid expansion part and the twelfth fluid expansion part.

3. The oral balloon of claim 2, wherein the fluid bag has an integrated shape formed over the eleventh fluid expansion part, the twelfth fluid expansion part, and the bridge part, and the fluid injection port is formed in the bridge part.

4. The oral balloon of claim 1, further comprising a fluid bag separation gap,
wherein, when the fluid bag is filled with fluid, the fluid bag has a shape restricted by the fluid bag separation gap.

5. The oral balloon of claim 1, further comprising an insertion guide part connected to a fluid expansion part that forms the fluid bag,
wherein an implant is formed in the insertion guide part, the implant being configured to insert the oral balloon into an oral cavity, and
the implant substantially maintains a shape of the oral balloon when the oral balloon is inserted into the oral cavity.

6. The oral balloon of claim 2, wherein a pocket having an inlet formed toward a central portion of the oral balloon is formed in each of the eleventh fluid expansion part and the twelfth fluid expansion part.

7. The oral balloon of claim 1, wherein ultrasonic energy or RF wave energy introduced to the fluid has a magnitude greater than that of ultrasonic energy or RF wave energy passing out of the fluid.

8. A method for installing the oral balloon of claim 1 in a space between a face and an oral cavity of a patient, the method comprising:
arranging a fluid bag into a mouth of the patient; and
expanding the fluid bag by injecting fluid through the fluid injection port after the arranging of the fluid bag.

9. A method for installing the oral balloon of claim 1 in a space between a face and an oral cavity of a patient, the method comprising:
expanding the fluid bag by injecting fluid through the fluid injection port; and
arranging the fluid bag into a mouth of the patient after the expanding of the fluid bag.

10. An oral balloon comprising:
a second chamber (220) into which fluid can be injected;
a first chamber (210) arranged to surround a portion of an edge of the second chamber;
a third chamber (230) filled with fluid; and
a fluid movement passage (240) configured to receive the fluid from the third chamber and provide the fluid to the second chamber,
wherein the first chamber is filled with fluid, and when pressure is applied to the third chamber, the fluid contained in the third chamber is supplied to the second chamber through the fluid movement passage to expand the second chamber.

11. The oral balloon of claim 10, wherein the first chamber and the second chamber are separated from each other, the first chamber and the third chamber are separated from each other, and the fluid movement passage and the second chamber are disposed between the first chamber and the third chamber.

12. The oral balloon of claim 10, wherein a first point (251) of a first surface of the second chamber and a second point (252) of a second surface of the second chamber are bonded to each other, and a three-dimensional shape of the second chamber, which is formed when the second chamber is expanded, is determined by the bonding of the first point and the second point.

13. The oral balloon of claim 10, wherein the fluid movement passage has one end connected to a portion of an edge of the second chamber, which is not surrounded by the first chamber, and the other end connected to a point on an edge of the third chamber.

14. A method for manufacturing the oral balloon of claim 10, the method comprising:
overlapping a first sheet (410) and a second sheet (420);
performing thermal bonding to form the first chamber, the second chamber, the third chamber, and the fluid movement passage except for the fluid injection part configured to inject fluid to the first chamber and the third chamber;
injecting fluid through the fluid injection part formed in each of the first chamber and the third chamber; and
performing thermal bonding to block the fluid injection part of the first chamber and the fluid injection part of the third chamber.

15. The method of claim 14, wherein the performing of the thermal bonding comprises thermally bonding a first point of the first sheet of the second chamber and a second point of the second sheet of the second chamber.
